# EUROPEAN PATENT APPLICATION

(11) **EP 2 371 303 A1**
(43) Date of publication of application: **05.10.2011**
(21) Application number: 11152567.1
(22) Date of filing: 28.01.2011
(51) Int. Cl.: A61B 17/128, A61B 17/122

(54) **Ligating apparatus**

(30) Priority: 29.03.2010 JP 2010076456
(71) Applicant: Fujifilm Corporation, Minato-ku Tokyo (JP)
(72) Inventor: Matsuoka, Yoshiaki, Kanagawa (JP); Iwasaka, Masayuki, Kanagawa (JP)
(74) Representative: Klunker . Schmitt-Nilson . Hirsch

(57) **Abstract**

A ligating-apparatus includes a clip, an operating wire, an inner sheath member and an outer sheath member. The clip ligates a body tissue with arms. The operating wire pulls the clip. The inner sheath member encloses the operating wire to support a clip holding member. The outer sheath member advances and retreats while covering the inner sheath member. The outer sheath member changes the degree of opening of the arms by causing each arm to abut on an inner peripheral surface thereof. A pair of arms includes first arm portion and a second arm portion connected continuously to the first arm portion via a bending portion. When the bending portion abuts against an inner peripheral surface end portion of the outer sheath member, the second arm portion is arranged at an inner side in a direction of a radius from an inner peripheral surface of the outer sheath member.

## Description

### BACKGROUND OF THE INVENTION

### 1. Technical Field

The present invention relates to a ligating apparatus for ligating a body tissue with a clip.

### 2. Description of Related Art

A ligating apparatus for ligating a body tissue with a clip is known (see, e.g., JP-A-2007-209775 and JP-A-2007-136128). The ligating apparatus of such a type is configured such that a distal end thereof is inserted from a forceps opening of an endoscope into a body cavity, that a body tissue is ligated by adjusting a clip to a desired position of the body tissue and then performing advancing/retreating operations of an operating wire to close the clip. A ligating apparatus described in JP-A-2007-209775 is configured such that a distal end thereof is drawn out of a body cavity while a clip once used to ligate a tissue is separated from an operating wire and remains placed in the body cavity, and that an unused clip taken out of a clip case which accommodates unused clips is attached to the distal end thereof. Consequently, a plurality of clips can successively be used by the same ligating apparatus to ligate body tissues in a body cavity.

However, with the configuration of the above ligating apparatus, after the arms of a clip are once closed, those of the clip cannot be opened again. That is, an operation of closing the arms of a clip can be performed only once. Thus, when a body tissue is clipped with a clip, it is necessary to carefully adjust the position of the clip so that the clip is positioned at a desired place and directed in a desired direction. Accordingly, a ligating operation of a clip requires skill.

A ligating apparatus described in JP-A-2007-136128 has a double sheath configuration in which an inner sheath member and an outer sheath member are arranged outside an operating wire. A distal-end-side part of the clip can be opened and closed by performing advancing/retreating operations of the outer sheath member. However, actually, a body tissue to be clipped is hidden by the distal end part of the outer sheath member. Accordingly, a body tissue cannot be ligated by simultaneously observing the body tissue.

### SUMMARY

An object of the invention is to provide a ligating apparatus that can freely perform operations of opening and closing a clip when performing an operation of ligating a body tissue, and that can re-grasp the tissue while observing the body tissue to be ligated.

The invention includes the following configuration.

A ligating apparatus for ligating a body tissue includes a clip, a clip holding member, an operating wire, an inner sheath member and an outer sheath member. The clip has a pair of freely openable/closable arms extended from a clip base portion and ligates a body tissue with the arms. The clip holding member has a communicating hole for causing the clip to be inserted thereinto. The operating wire performs an operation of pulling the clip into the communicating hole. The inner sheath member is configured to be extended to enclose the operating wire and supports the clip holding member at an end of the inner sheath member. The outer sheath member is arranged to advance and retreat while covering the inner sheath member. The outer sheath member changes a degree of opening of the pair of arms by causing the pair of arms to abut on an inner peripheral surface of the outer sheath member. Each of the pair of arms includes a first arm portion and a second arm portion which is connected continuously to the first arm portion via a bending portion. The first and second arm portions are arranged from a base portion side in order. When the outer sheath member is moved close to a clip side along the inner sheath member, so that the bending portions abuts against an inner peripheral surface end portion of the outer sheath member, the second arm portion is arranged at an inner side in a direction of a radius from the inner peripheral surface of the outer sheath member.

The ligating apparatus according to the invention, in which a plurality of clips are sequentially replaceable, can freely perform opening/closing of a clip when performing an operation of ligating a body tissue, and can re-grasp the tissue by a simple operation.

### BRIEF DESCRIPTION OF THE DRAWINGS

FIG. 1 is a partly cutaway plan view of a ligating apparatus, which is a diagram for illustrating an embodiment of the invention.
FIG. 2 is a cross-sectional view of a distal end portion of the ligating apparatus illustrated in FIG. 1.
FIG 3 is a perspective view of a clip unit illustrated in FIG. 2.
FIG 4A is a cross-sectional view of a primary part of the ligating apparatus at the retreat of an outer sheath member. FIG. 4B is a cross-sectional view of the primary part at the running-out of the outer sheath member.
FIG. 5A is a cross-sectional view illustrating a primary part of the ligating apparatus at the position-adjustment of a body tissue. FIG. 5B is a cross-sectional view illustrating the primary part at the clipping of the body tissue with a clip. FIG. 5C is a cross-sectional view illustrating the primary part at the pull-in of the clip. FIG 5D is a cross-sectional view illustrating the primary part at the separation of the clip.
FIG 6A is a plan view illustrating a modification in which bending portions are formed to have a narrow width. FIG 6B is a side view illustrating the modification illustrated in FIG 6A.
FIG 7 is a side view illustrating a modification in which bending portions are formed to have a thin thickness.

### DETAILED DESCRIPTION OF EMBODIMENTS OF THE INVENTION

Hereinafter, embodiments of the invention are described with reference to the drawings.

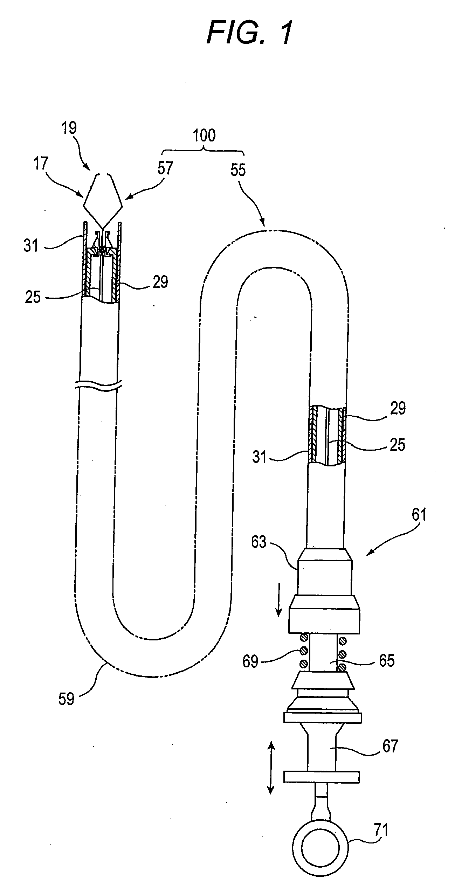

FIG. 1 is a partly cutaway plan view of a ligating apparatus, which is a diagram for illustrating an embodiment of the invention.

A ligating apparatus 100 is configured by roughly including an operating device 55, and a clip 19. The operating device 55 is configured to include an inserting portion 59 and a proximal side operating portion 61. The inserting portion 59 is configured to include an outer sheath member 31, an inner sheath member 29 inserted into the outer sheath member 31, and an operating wire 25 inserted into the inner sheath member 29. The proximal side operating portion 61 is configured to include an outer sheath connecting element 63 fixed to the proximal side of the outer sheath member 31, an operating portion body 65 for performing advancing/retreating operations of the inner sheath member 29 with respect to the outer sheath member 31, a slider portion 67 for performing advancing/retreating operations of the operating wire 25 with respect to the inner sheath member 29, a slider spring 69 provided between the outer sheath connecting element 63 and the slider portion 67, and a finger hook ring 71.

The operating device 55 is such that the inner sheath member 29 protrudes from the outer sheath member 31 when the operating portion body 65 is pushed towards the distal end thereof. When the slider portion 67 is pushed to the distal end side thereof, the operating wire 25 moves to the direction of the distal end thereof in the inner sheath member 29. The operating wire 25 is formed of a metal twisted wire made of stainless steel, NiTi-alloy or the like having appropriate elasticity. The clip 19 is detachably attached to an inner sheath distal-end portion 27 in which the operating wire 25 can be advanced and retreated.

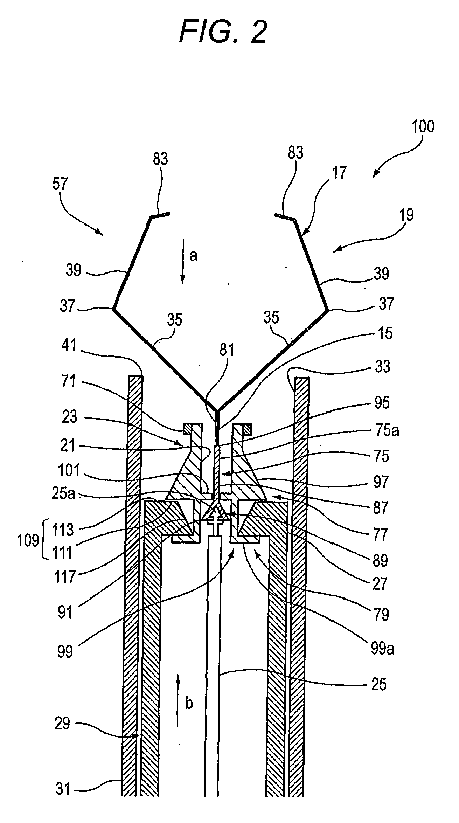

FIG. 2 is a cross-sectional view of the distal end portion of the ligating apparatus 100 illustrated in FIG 1.

The clip 19 includes openable/closable arms 17 for ligating a body tissue, a clip connecting member 75, a clip holding member 23, a first movement restricting portion 77, and a second movement restricting portion 79. The arms 17 are formed like a pair of open leg elements by outwardly bending each metal plate member made of metal, such as stainless steel, having appropriate spring properties at a central portion thereof. An intersecting portion 81 is formed at a back end of each arm 17. A tissue grasping portion 83 serving as a claw portion is formed at the distal end portion thereof. In the present specification, a side in a direction in which the ligating apparatus 100 is inserted is referred as a front side. The side opposite to the front side is referred to as a back side.

The clip connecting member 75 is continuously connected to a clip base portion 15 serving as a back end of the intersecting portion 81. The clip connecting member 75 is such that a connecting member distal-end portion 75a serving as a distal end of the clip connecting member 23 is connected to the clip base portion 15. The clip connecting member 75 is made of a high-strength resin material, e.g., nylon (trademark) and formed cylindrically A fragile portion 95 which will be described below is provided between the connecting member distal-end portion 75a and the clip base portion 15. That is, the clip 19 and the clip connecting member 75 are configured to be connected to each other via the fragile portion 95. A wire connecting portion 89 is provided in the connecting member base portion 87 of the clip connecting member 75. The wire connecting portion 89 is formed as a conical diameter-increased portion 91 formed by connecting the top part of a hollow conical body continuously to the connecting member base portion 87.

The conical diameter-increased portion 91 serves to spread an elastically bending leg-element which will be described below. An engaging hole which will be described below is bored in a back-end surface of the conical diameter-increased portion 91. The engaging hole is such that an arrowhead-like distal-end portion 25a provided at the distal end portion of the operating wire 25 can detachably be inserted thereinto. The arrowhead-like distal-end portion 25a is caught in the engaging hole in the conical diameter-increased portion 91 by barb parts provided at the back end parts of the conical diameter-increased portion 91, so that the arrowhead-like distal-end portion 25a is restricted from being disengaged therefrom. Accordingly, the clip 19 is connected to the operating wire 25 by the clip connecting member 75 and the arrowhead-like distal-end portion25a caught in the conical diameter-increased portion 91.

A disengagement restricting force generated by the arrowhead-like distal-end portion 25a is set to be larger than the strength of the fragile portion 95. This is because the wire 25 is prevented from being disengaged from the arrowhead-like distal-end portion 25a before the fragile portion 95 is fractured. When the clip 19 is separated from the clip connecting member 75 by fracturing the fragile portion 95, the clip connecting member 75 is connected to the operating wire 25 and taken out of the body cavity while the arrowhead-like distal-end portion 25a remains caught therein. The clip connecting member 75 taken out therefrom is disengaged from the arrowhead-like distal-end portion 25a with a finger or the like by a predetermined extracting force.

The clip holding member 23 includes a conical body portion 97 provided at a front part thereof and an elastically bending leg-element 99 provided posterior to the conical body portion 97. The clip holding member 23 also includes a communicating hole 21 extending in a direction along an axis line of the conical body portion 97 to pass the clip 17 and the clip connecting member 75 therethrough. The clip holding member 23 is formed by, e.g., performing injection molding on a resin. A narrowing portion 101 is provided in the communicating hole 21. A more front portion of the clip connecting member 23 than the conical diameter-increased portion 91 is inserted through the narrowing portion 101. That is, the conical diameter-increased portion 91 abuts against the narrowing portion 101 to thereby restrict the clip connecting member 75 from being slipped off more frontwardly therefrom. On the other hand, the narrowing portion 101 allows the backward extraction of a connecting member distal-end portion 75a.

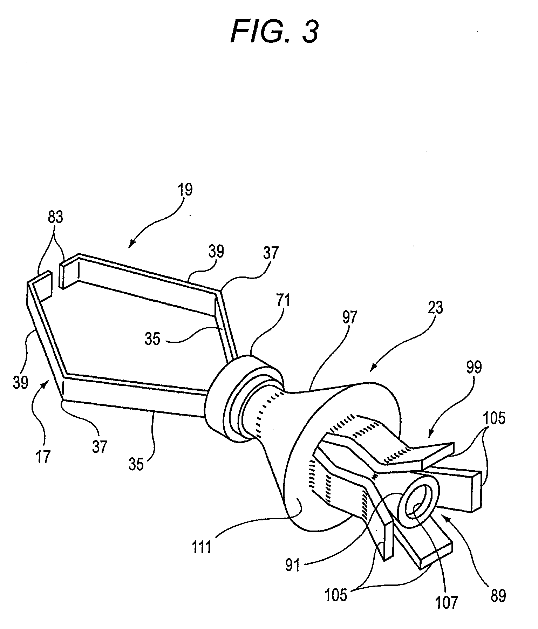

FIG 3 is a perspective view of a clip unit illustrated in FIG 2.

An abutment ring 71 is formed at the front part of the conical body portion 97 coaxially with the communicating hole 21. The abutment ring 71 abuts against a separating wall portion serving as a stopper portion of a clip case (not shown). The elastically bending leg-element 99 protruding backwardly from a back end surface of the conical body portion 97 is such that, e.g., four leg portions 105 are radially arranged. Each leg portion 105 is formed into an outwardly bent shape. The elastically bending leg-element 99 is such that the leg portions 105 thereof are radially arranged, so that a substantially conical central space is formed. In the central space, the conical diameter-increased portion 91 is arranged. An engaging hole 107 through which the above arrowhead-like distal-end portion 25a is detachably inserted is opened in the back end surface of the conical diameter-increased portion 91.

That is, the conical diameter-increased portion91 arranged in the central space is configured to spread out, when pressed by the operating wire 25, the leg portions 105 to thereby advance. The leg portions 105 spread out by the advancement of the conical diameter-increased portion 91 are opened by opening the back end parts of the leg portions 105 due to the fact that the bent part of each leg portion 105 is outwardly pressed. The back end of each opened leg portion 105 is caught in a back part of an inner sheath distal-end portion, which will be described below.

Each of the outer sheath member 31 and the inner sheath member 29 can be configured by, e.g., a coil sheath having flexibility, which is obtained by closely coiling a spring-property material. The outer sheath member 31 is arranged to cover the outer periphery of the inner sheath member 29. The inner sheath member 29 freely advances and retreats in the outer sheath member 31. That is, the outer sheath member 31 is arranged to freely advance and retreat with respect to the inner sheath member 29. The outer sheath member 31 works to change the degree of opening of the arms 17 by causing the arms 17 to abut on the inner peripheral surface 33 of the outer sheath member 31. The inner sheath member 29 is extended to enclose the operating wire 25. The inner sheath member 29 supports the clip holding member 23 in the inner sheath distal-end portion 27. The operating wire 25 inserted in the inner sheath portion 29 performs an operation of pulling the clip connecting member 75 to thereby cause the clip 19 to freely be operated in an advancing/retreating direction.

Turning back to FIG 2, the ligating apparatus 100 includes the first movement restricting portion 77 and the second movement restricting portion 79, which restrict the movement of the clip holding member 23 with respect to the inner sheath member 29. The first movement restricting portion 77 restricts the movement of the clip holding member 23 to the axially inner side (i.e., in a direction designated by arrow a) from the axially outer side of the inner sheath member 29. The second movement restricting portion 79 restricts the movement of the clip holding member 23 to the axially outer side (i.e., in a direction designated by arrow b) from the axially inner side of the inner sheath member 29.

The first movement restricting portion 77 is configured as catching surfaces 109 abutting each other, which are respectively formed on the clip holding member 23 and the inner sheath member 29. The catching surfaces 109 of the clip holding member 23 and the inner sheath member 29 abut against each other. Consequently, the movement of the clip holding member 23 can surely be restricted.

More specifically, the catching surfaces 109 are respectively formed as a bottom surface 111 of the conical body portion 97 formed in the clip holding member 23 and an inner sheath bearing surface 113 formed on the inner sheath distal-end portion 27. Consequently, the conical body portion 97 of the clip holding member 23 and the inner sheath bearing surface 113 of the inner sheath member 29 are caused to abut against each other, so that the movement of the clip holding member 23 in the direction of arrow a is restricted.

The second movement restricting portion 79 is formed as the elastically bending leg-element 99 extended towards the inner sheath member 29 from the communicating hole 21 of the clip holding member 23. The elastically bending leg-element 99 is spread out when the clip connecting member 75 is inserted into the communicating hole 21. Accordingly, a bearing surface forming portion 117 of the inner sheath member 29 is sandwiched between a leg distal-end portion 99a and the bottom surface 111 of the conical body portion 97. That is, the inner sheath member 29 and the clip 19 can be fixed to each other by spreading out the elastically bending leg-element 99.

When the clip 19 is taken out of the communicating hole 21 of the clip holding member 23, the elastically bending leg-element 99 is elastically returned to a state before the leg 99 is spread. That is, the engagement between the inner sheath member 29 and the clip holding member 23, or the release of the engagement therebetween can be performed depending upon whether the insertion of the elastically bending leg-element 99 into the communicating hole 21 is performed or not.

The clip 19 is inserted into the communicating hole 21 of the clip holding member 23 by pulling the operating wire 25. Thus, the degree of opening of the arms 17 is fixed. For example, when the clip 19 is inserted into the communicating hole 21 by pulling the operating wire 25, a back part of the clip 19 is narrowed at the front-part opening edge of the communicating hole 21. The arms 17 are fixed in a closed state.

The clip connecting member 75 includes the fragile portion 95 which can be fractured when the operating wire 25 is further pulled after the clip 19 is inserted into the communicating hole 21 of the clip holding member 23 by pulling the operating wire 25, as described above. The fragile portion 95 is formed as a fracturing portion to have a small diameter. The size of a cross-section of the fragile portion 95 is set so that the fragile portion 95 is fractured when a rupture force of about 20 newtons (N) to about 60 N is applied thereto. When the fragile portion 95 is fractured, the clip 19 and the operating wire 25 are separated from each other. That is, the clip 19 can be separated from the operating wire 25 in a state, in which a body tissue is grasped by the clip 19, by pulling the operating wire 25.

The clip connecting member 75 includes the above conical diameter-increased portion 91 for spreading out the elastically bending leg-element 99. The arrowhead-like distal-end portion 25a of the operating wire 25 is detachably inserted into the conical diameter-increased portion 91. The formation of the conical diameter-increased portion 91 in the clip connecting member 75 negates the diameter-increasing effect of the conical diameter-increased portion 91 on the elastically bending leg-element 99 after the clip connecting member 75 is taken out of the clip holding member 23. Consequently, the elastically bending leg-element 99 is elastically restored to automatically release the connection between the clip holding member 23 and the inner sheath member 29. Accordingly, the clip holding member 23 is indwelt in the body together with the clip 19 clipping a body tissue. The conical diameter-increased portion 91 is such that the arrowhead-like distal-end portion 25a of the operating wire 25 can easily be connected thereto by being detachably snap-inserted or that after separated from the clip 19, the conical diameter-increased portion 91 can be detached from and attached to the arrowhead-like distal-end portion 25a.

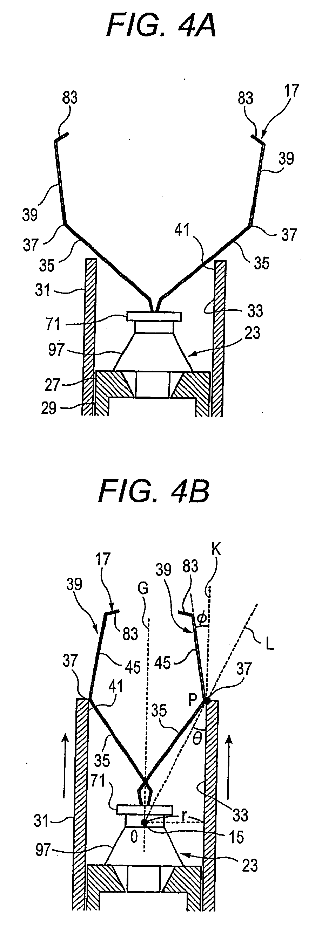

FIG 4A is a cross-sectional view of a primary part of the ligating apparatus 100 at the retreat of the outer sheath member 31. FIG 4B is a cross-sectional view of the primary part at the paying-out of the outer sheath member 31.

The outer sheath member 31 changes the degree of opening of the arms 17 by causing each arm 17 to abut on an associated inner peripheral end portion 41. A pair of arms 17 is formed such that each arm 17 includes a first arm portion 35, and a second arm portion 39 connected continuously to the first arm portion 35 via a bending portion 37, which are arranged in this order from the side of the base portion 15. The arms 17 are such that when the outer sheath member 31 is made to move along the inner sheath member 29 to the clip side so that each bending portion 37 abuts against the inner peripheral surface end portion 41 of the outer sheath member 31, as illustrated in FIG 4B, each second arm portion 39 is arranged radially inwardly from the inner peripheral surface 33 of the outer sheath member 31.

That is, when each bending portion 37 coincides with the inner peripheral surface end portion 41, the associated second arm portion 39 is inclined to an imaginary extension plane K of the inner peripheral surface end portion 41 in a direction in which the distal-end-side part of the second arm portion 39 approaches an axis line G by an angle φ formed by the imaginary extension plane K and the second arm 39 extending from the associated bending portion 37 serving as a reference. In a structure in which the bending portion 37 does not exist, a segment L passing through the clip base portion 15 and the inner peripheral surface end portion 41 is extended while the segment L is inclined outwardly to the imaginary extension plane K by an angle θ. That is, the second arm portion 39 protrudes from the bending portion 37 along the segment L. Thus, the distance between the second arm portions 39, 39 increases towards the distal end of each second arm portion 39. Accordingly, the clip 19 is not completely closed.

Each second arm portion 39 includes a tissue grasping portion 83 serving as a claw portion formed at the distal end thereof for grasping a body tissue, and an elongated piece portion 45 connected continuously to the tissue grasping portion 83 and the bending portion 37. When a body tissue is ligated, the body tissue can be grasped without slipping. The body tissue grasped by the elongated piece portions 45, 45 can be held by being pinched.

In a state in which each arm 17 (particularly, each first arm portion 35) is not covered with the outer sheath member 31, the arms 17 are shaped such that the distance between the arms 17 (particularly, the bending portions 37) is larger than the inside diameter of the outer sheath member 31. Accordingly, when the outer sheath member 31 covers and narrows the arms 17 a reaction force acting in a direction in which the arms 17 are spread out. Consequently, the advancement/retreat of the outer sheath member 31 enables the opening/closing of the arms 17. After each arm 17 abuts against the inner peripheral surface end portion 51 of the outer sheath member 31, the bending portions 37 reach the inner peripheral surface end portions 41 while the arms 17 slide on the inner peripheral surface end portion 41 of the outer sheath member 31 by causing the outer sheath member 31 to pay out. Thus, a clipping distance 119 can be adjusted by being changed by appropriately increasing or decreasing a clipping force when the length of the tissue grasping portion 83 is determined.

Thus, the arms 17 are opened and closed by the outer sheath member 31 and can temporarily hold a target tissue. If a ligating position is checked in a state in which the tissue is temporarily held, and no problems are detected, next, the clip holding member 23 is caused to advance to the arms 17 to perform main ligation. At that time, the inside diameter of the clip holding member 23 is far smaller than that of the outer sheath member 31. Accordingly, a large amount of an operating force is necessary for performing the main ligation on the already closed arms 17 with the clip holding member 23. It is difficult for an operator to perform such an operation. In addition, buckling of the inner sheath member 29 may occur. Thus, preferably, the bending stiffness of each bending portion 37 is set to be lower than that of the entire arms 17. At a temporarily holding stage, it is important to check the positional relationship thereamong. Accordingly, even when the bending stiffness of the bending portion 37 is reduced, no major problems are caused. At a main ligation stage, the inside diameter of the clip holding member 23 is small. The tissue grasping portions 83 of the clip 19 substantially abut against each other via the target tissue to maintain the distance between the second arm portions 39 to be equal to or more than the inside diameter of the clip holding member 23. Consequently, a sufficient ligating force is generated.

Next, an operation of the ligating apparatus 100 having the above configuration is described hereinafter.

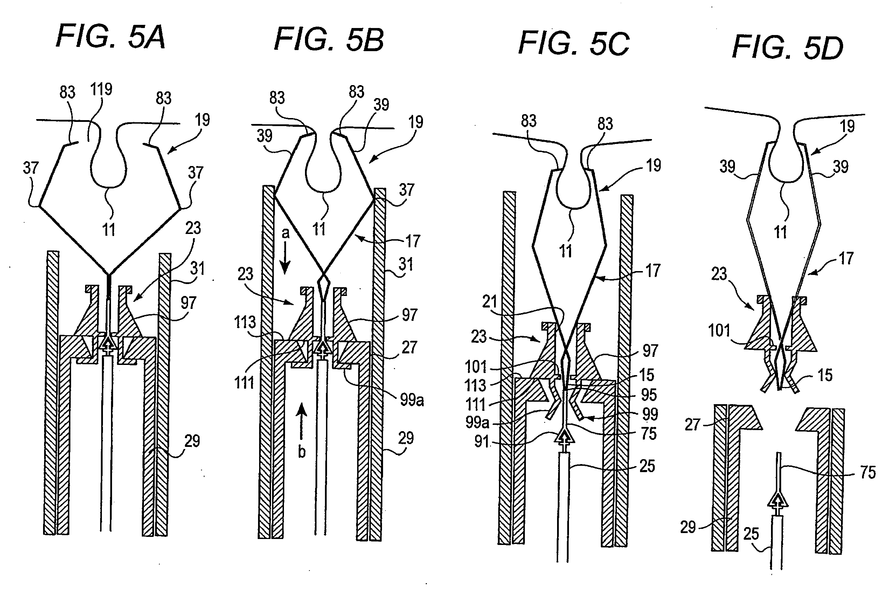

FIG. 5A is a cross-sectional view illustrating a primary part of the ligating apparatus at the position-adjustment of a body tissue. FIG 5B is a cross-sectional view illustrating the primary part the clipping of the body tissue with a clip. FIG 5C is a cross-sectional view illustrating the primary part at the pull-in of the clip. FIG 5D is a cross-sectional view illustrating the primary part at the separation of the clip.

In order to ligate the body tissue 11 with the clip 19, the distal end part of the inserting portion 59 of the operating device 55 is introduced into the body cavity via a channel of an endoscope (not shown) preliminarily inserted into the body cavity. Then, the distal end of the inserting portion 59 is introduced to a target part while the inside of the body cavity is observed with the endoscope. When it is confirmed that the distal end of the inserting portion 59is introduced to the target part, the operating portion body 65 is pressed thereinto. When the operating portion body 65 is pressed thereinto, the inner sheath member 29 advances in the outer sheath member 31. Consequently, as illustrated in FIG 5A, the clip 19 protrudes from the outer sheath member 31. The leg-element of the protruded clip 19 is opened by an elastically restoring force to form a clipping gap 119 between the tissue grasping portions 83, 83.

The positioning of the clip 19 is performed such that the body tissue 11 is arranged in the clipping gap 119. When a force for pressing the operating portion body 65 is loosened in a state in which the position of the tissue grasping portion 83 is optimal, the outer sheath member 31 advances to the inner sheath member 29 by the elastically restoring force of the slider spring 69 (see FIG 1). Consequently, as illustrated in FIG 5B, the arms 17 of the clip 19 are pulled into the outer sheath member 31. Thus, the body tissue 11 is grasped with the tissue grasping portions 83. At that time, because each arm 17 is provided with the bending portion 37, the second arm portions 39 provided at a more distal end side than the bending portions 37are exposed from the outer sheath member 31 even when the bending portions 37 are covered with the outer sheath member 31. Accordingly, the clip 19 can grasp a body tissue at a desired position while the body tissue is visually recognized. Consequently, operability is enhanced.

If a grasping position at which the tissue grasping portions 83 grasp the body tissue 11 is changed at that time, the inner sheath member 29 is protruded again by pressing the operating portion body 65. Thus, the diameter reducing effect of the outer sheath member 31 on each arm 17 is canceled. That is, the ligating apparatus 100 is returned to the state illustrated in FIG. 5A, in which the tissue grasping portions 83, 83 are opened so that the grasping position can iteratively be changed. Thus, in the ligating apparatus 100, the degree of opening of the distal ends of the arms 17 can iteratively be changed by the advancement/retreat of the outer sheath member 31. The regrasping of the body tissue 11 can freely be performed.

These are implemented by holding the clip 19 at the inner sheath distal-end portion 27. The clip 19 is such that the bottom surface 111 of the conical body portion 97 abuts on the inner sheath bearing surface 113, and that the movement in the direction of arrow a of the clip holding member 23 is restricted. A leg distal-end portion 99a is caught on the back surface of the inner sheath distal-end portion 27. Thus, the movement in the direction of arrow b of the clip holding member 23 is restricted. That is, the clip 19 is held by clipping the inner sheath distal-end portion 27.

When the grasping position at which the tissue 11 is grasped by the tissue grasping portions 83 is definitely determined, the slider portion 67 is pulled. Then, as illustrated in FIG 5C, the clip connecting member 75 is pulled via the operating wire 25. The clip 19 pulled with the operating wire 25 is such that the bottom surface 111 of the conical body portion 97 abuts against the inner sheath bearing surface 113 to thereby restrict the retreat of the clip holding member 23. On the other hand, the clip 19 connected continuously to the clip connecting member 75 is pulled into the communicating hole 21 while the tissue grasping portions 83 grasp the body tissue 11. Thus, a larger grasping force acts upon the tissue grasping portions 83.

The clip connecting member 75 pulled with the operating wire 25 pulls the fragile portion 95 and the clip base portion 15 into the narrowing portion 101. Simultaneously with this, the conical diameter-increased portion 91 falls off the elastically bending leg-element 99. Thus, the elastically bending leg-element 99 is narrowed. The narrowing of the elastically bending leg-element 99 cancels the catching of the clip 19 by the leg back-end portions 99a. However, a pulling force due to the operating wire 25 acts upon the entire clip 19. Thus, the clip 19 doesn't fall off the inner sheath distal-end portion 27. When the clip base portion 15 passes through the narrowing portion 101, a reaction force exerted on the arms 17 from the opening edge of the communicating hole 21 increases. Accordingly, a pulling resistance force increases. If the pulling resistance force exceeds a predetermined value, the fragile portion 95 is fractured. Thus, as illustrated in FIG 5D, the clip base portion 15 and the clip connecting member 75 are separated from each other.

The clip 19 separated from the clip connecting member 75 is such that the clip base portion 15 is opened in rear of the narrowing portion 101 and restricts the clip 19 from slipping off frontwardly Accordingly, the clip 19 retains a state in which the clip 19 grasps the body tissue 11. The clip 19 whose arms 17 are restricted by the clip holding member 23 from being opened is indwelt in the body, together with the clip holding member 23, as it is.

Thus, the ligating apparatus 100 is such that the clip holding member 23 is restricted from being axially moved with respect to the inner sheath member 29. Consequently, the ligating apparatus 100 can prevent the slip-off of the clip 19 and the relative movement between the operating wire 25 and the inner sheath member 29 from occurring when the arms 17 of the clip 19 are opened and closed by the outer sheath member 31. Accordingly, the handleability of the ligating apparatus 100 is improved.

Accordingly, according to the above ligating apparatus 100 in which a plurality of clips 19 can be sequentially replaced, when an operation of ligating the body tissue 11 is performed, operations of opening and closing the clip 19 can freely be performed. In addition, the regrasping of the body tissue 11 can be performed by a simple operation. Even when the second arm portion 39 is placed outside the outer sheath member 31, the tissue grasping portion 83 can be brought into a closed state. Thus, the visibility of the body tissue 11 at the ligation thereof can be enhanced.

Next, a modification of the ligating apparatus 100 of the above configuration is described below.

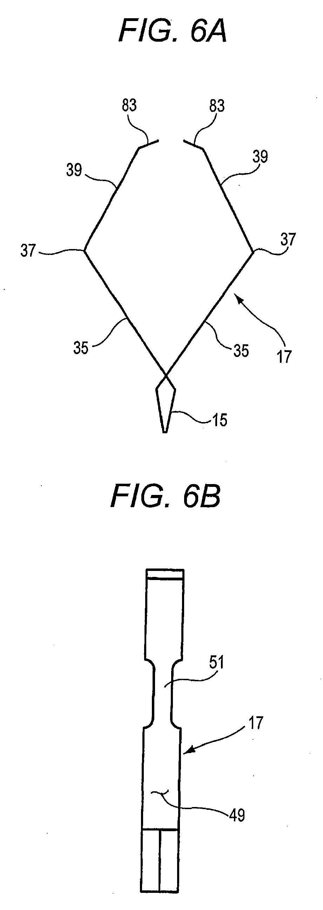

FIG 6A is a plan view illustrating a modification in which the bending portions 37 are formed to have a narrow width. FIG 6B is a side view illustrating the modification illustrated in FIG 6A.

This modification is such that each bending portion 37 includes a narrow area 51 formed by cutting away a side surface 49 of an associated arm 17. The narrow area 51 can be formed either both or one of the side surface 49.

This modification is such that a cutout is provided in the bending portion 37 of each arm 17. Consequently, the bending stiffness can easily be reduced.

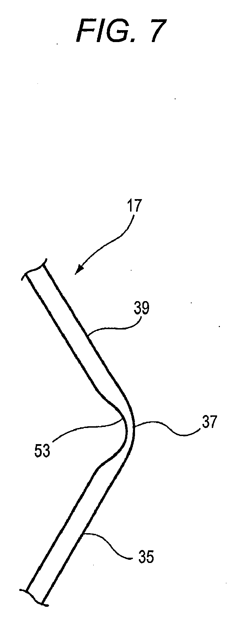

FIG. 7 is a side view illustrating a modification in which bending portions are formed to have a thin thickness.

This modification is such that the bending portion 37 includes a thin area 53 configured by setting the thickness of the arm 17 to be thin, as compared with the thickness of the periphery thereof.

According to this modification, the bending portion 37 of the arm 17 is formed to be thin. Thus, the bending stiffness can easily be lowered. In addition, substantially no irregularities are caused in the outer shape of the clip 19. The shape of the clip 19 is such that when the clip is indwelt in the body, impact on the body tissue is low.

In addition, each arm 17 can be made of a metal material. The bending portions 37 can be reduced in the bending stiffness by thermal treatment.

According to this modification, the bending portions 37 of the arms 17 can locally be changed in properties by thermal treatment. Thus, the bending stiffness of the bending portions 37 can be reduced without changing the outer shape and the cross-sectional shape thereof. Consequently, similarly, impact on the body tissue can be reduced when the clip is indwelt in the body.

Thus, the invention is not limited to the above embodiments. The invention is intended to be susceptible to modifications and applications made by those skilled in the art based on the descriptions of the specification and known technology. The modifications and the applications are included within a scope of protection.

As described above, the following items are disclosed in the present specification.
(1) A ligating apparatus for ligating a body tissue includes a clip, a clip holding member, an operating wire, an inner sheath member and an outer sheath member. The clip has a pair of freely openable/closable arms extended from a clip base portion and ligates a body tissue with the arms. The clip holding member has a communicating hole for causing the clip to be inserted thereinto. The operating wire performs an operation of pulling the clip into the communicating hole. The inner sheath member is configured to be extended to enclose the operating wire and supports the clip holding member at an end of the inner sheath member. The outer sheath member is arranged to advance and retreat while covering the inner sheath member. The outer sheath member changes a degree of opening of the pair of arms by causing the pair of arms to abut on an inner peripheral surface of the outer sheath member. Each of the pair of arms includes a first arm portion and a second arm portion which is connected continuously to the first arm portion via a bending portion. The first and second arm portions are arranged from a base portion side in order. When the outer sheath member is moved close to a clip side along the inner sheath member, so that the bending portions abuts against an inner peripheral surface end portion of the outer sheath member, the second arm portion is arranged at an inner side in a direction of a radius from the inner peripheral surface of the outer sheath member.
   According to this ligating apparatus, each arm is provided with the bending portion. Thus, even when the bending portions are covered with the outer sheath member, the arms provided at a more distal end side than the bending portions are exposed from the outer sheath member. Accordingly, the clip can grasp a body tissue at a desired position while the body tissue is visually recognized. Consequently, operability is enhanced. The degree of opening of the distal ends of the arms can iteratively be changed by the advancement/retreat of the outer sheath member. The regrasping of the body tissue can freely be performed.
(2) The ligating apparatus according to (1), the second arm portion includes a claw portion and an elongated piece portion. The claw portion is formed at an end of an associated one of the pair of arms to grasp the body tissue. The elongated piece portion is continuously connected between the claw portion and the bending portion.
   According to this ligating apparatus, when a body tissue is ligated, the body tissue can be grasped without slipping off. The body tissue grasped by the elongated piece portions can be held by being pinched.
(3) The ligating apparatus according to (1) or (2), the pair of arms are configured so as to have a distance between the pair of arms is larger than an inside diameter of the outer sheath member.
   According to this ligating apparatus, after each arm abuts against the inner peripheral surface end portion of the above outer sheath member, the bending portions reach the inner peripheral surface end portions while the arms slide on the inner peripheral surface end portion of the outer sheath member by causing the outer sheath member to pay out.
(4) The ligating apparatus according to any one of (1) to (3), bending stiffness corresponding to a bending operation for changing a degree of opening of the bending portions of the clip is set to be lower than that of the entire pair of arms.
   According to this ligating apparatus, the bending stiffness is reduced. Thus, when a body tissue is grasped, a relatively large tolerance of the thickness of a graspable body tissue is provided. That is, body tissues ranging from thin ones to thick ones can surely be grasped. In addition, body tissues are prevented from being grasped by excessively being compressed. Consequently, the tissues grasped by the arms can be protected.
(5) The ligating apparatus according to (4), each of the bending portions has a narrow area which is formed by cutting out a side surface of one of the pair of arms.
   According to this ligating apparatus, the bending stiffness of the bending portion of each arm can be reduced by providing a cutout therein.
(6) The ligating apparatus according to (4) or (5), each of the bending portions has a thin area of which thickness is set to be smaller than that of periphery of the thin area.
   According to this ligating apparatus, the bending stiffness of each bending portion can be reduced by decreasing the thickness thereof.
(7) The ligating apparatus according to any one of (4) to (6), each of the pair of arms is made of a metal material. The bending stiffness of each of the bending portions is reduced by thermal treatment.
   According to this ligating apparatus, the bending stiffness of the bending portion of each arm can be reduced, without changing the cross-sectional shape of the bending portion, by locally changing the property thereof through thermal treatment.

## Claims

1. A ligating apparatus for ligating a body tissue, comprising:
a clip that has a pair of freely openable/closable arms extended from a clip base portion, and that ligates a body tissue with the arms;
a clip holding member that has a communicating hole for causing the clip to be inserted thereinto;
an operating wire that performs an operation of pulling the clip into the communicating hole;
an inner sheath member that is configured to be extended to enclose the operating wire and that supports the clip holding member at an end of the inner sheath member; and
an outer sheath member that is arranged to advance and retreat while covering the inner sheath member,
wherein the outer sheath member changes a degree of opening of the pair of arms by causing the pair of arms to abut on an inner peripheral surface of the outer sheath member,
wherein each of the pair of arms includes a first arm portion and a second arm portion which is connected continuously to the first arm portion via a bending portion, the first and second arm portions being arranged from a base portion side in order, and
wherein when the outer sheath member is moved close to a clip side along the inner sheath member, so that the bending portions abuts against an inner peripheral surface end portion of the outer sheath member, the second arm portion is arranged at an inner side in a direction of a radius from the inner peripheral surface of the outer sheath member.

2. The ligating apparatus according to claim 1,
wherein the second arm portion includes a claw portion and an elongated piece portion, wherein the claw portion is formed at an end of an associated one of the pair of arms to grasp the body tissue, and
wherein the elongated piece portion is continuously connected between the claw portion and the bending portion.

3. The ligating apparatus according to claim 1 or 2,
wherein the pair of arms are configured so as to have a distance between the pair of arms is larger than an inside diameter of the outer sheath member.

4. The ligating apparatus according to any one of claims 1 to 3,
wherein bending stiffness corresponding to a bending operation for changing a degree of opening of the bending portions of the clip is set to be lower than that of the entire pair of arms.

5. The ligating apparatus according to claim 4,
wherein each of the bending portions has a narrow area which is formed by cutting out a side surface of one of the pair of arms.

6. The ligating apparatus according to claim 4 or 5,
wherein each of the bending portions has a thin area of which thickness is set to be smaller than that of periphery of the thin area.

7. The ligating apparatus according to any one of claims 4 to 6,
wherein each of the pair of arms is made of a metal material, and
wherein the blending stiffness of each of the bending portions is reduced by thermal treatment.
